# EUROPEAN PATENT SPECIFICATION

(11) **EP 4 135 641 B1**
(45) Date of publication and mention of the grant of the patent: **28.10.2026**
(21) Application number: 21715978.9
(22) Date of filing: 29.03.2021
(51) Int. Cl.: A61F 13/02, A61F 13/15

(54) **MEDICAL ADHESIVE ARTICLES HAVING A LOW EFFECTIVE MODULUS OF ELASTICITY**
MEDIZINISCHE KLEBEARTIKEL MIT NIEDRIGEM ELASTIZITÄTSMODUL
ARTICLES ADHÉSIFS MÉDICAUX AYANT UN MODULE D'ÉLASTICITÉ EFFICACE FAIBLE

(30) Priority: 13.04.2020 US 202063009075 P
(43) Date of publication of application: 22.02.2023
(73) Proprietor: Solventum Intellectual Properties Company, Eagan, MN 55121 (US)
(72) Inventor: SHERMAN, Audrey A., Saint Paul, Minnesota 55133-3427 (US); LAWSON, Del R., Saint Paul, Minnesota 55133-3427 (US); GOLD, Anne C. F., Saint Paul, Minnesota 55133-3427 (US); GUTTMANN, Silvia G. B., Saint Paul, Minnesota 55133-3427 (US); CORRIGAN, Thomas R. J., Saint Paul, Minnesota 55133-3427 (US); SEDLOCK, Carole, Saint Paul, Minnesota 55133-3427 (US); VOELKER, Corey D., Saint Paul, Minnesota 55133-3427 (US)
(74) Representative: Vossius & Partner Patentanwälte Rechtsanwälte mbB
(86) International application number: PCT/IB2021/052591
(87) International publication number: WO 2021/209846

(56) References cited:
- EP-A1- 0 059 049
- US-A- 3 085 024
- US-A1- 2002 193 724
- US-A1- 2010 036 346
- US-A1- 2011 024 940

## Description

### Field of the Disclosure

The current disclosure relates to medical adhesive articles suitable for long wear, having a low effective modulus of elasticity.

### Background

A wide range of adhesive articles are used in medical applications. These adhesive articles include gels used to attach electrodes and other sensing devices to the skin of a patient, a wide range of tapes to attach medical devices to a patient, and adhesive dressings used to cover and protect wounds.

Many of the adhesive articles use pressure sensitive adhesives. Pressure sensitive adhesives are well known to one of ordinary skill in the art to possess certain properties at room temperature including the following: (1) aggressive and permanent tack, (2) adherence with no more than finger pressure, (3) sufficient ability to hold onto an adherend, and (4) sufficient cohesive strength to be removed cleanly from the adherend. Materials that have been found to function well as pressure sensitive adhesives are polymers designed and formulated to exhibit the requisite viscoelastic properties resulting in a desired balance of tack, peel adhesion, and shear strength.

The most commonly used polymers for preparation of pressure sensitive adhesives are natural rubber, synthetic rubbers (e.g., styrene/butadiene copolymers (SBR) and styrene/isoprene/styrene (SIS) block copolymers), various (meth)acrylate (e.g., acrylate and methacrylate) copolymers, and silicones. US2002193724 discloses examples of adhesive articles.

### Summary

The current disclosure relates to medical adhesive articles suitable for long wear having a low effective modulus of elasticity, as well as methods for preparing these medical adhesive articles. Also disclosed are medical constructions prepared using the medical adhesive articles.

In some embodiments, the medical adhesive articles comprise a continuous layer of adhesive with a first major surface and a second major surface, and a substrate layer with a first major surface and the second major surface, where the first major surface of the substrate layer is in contact with the second major surface of the adhesive layer. The substrate layer comprises a plurality of cuts, wherein the plurality of cuts is arrayed in a pattern. The pattern may be random, or the the pattern may be arrayed along at least one axis. The cuts are gaps, but the gaps are not visible to the naked eye when the adhesive article is in an unstressed state, and at least some of the cuts become gaps that are visible to the naked eye when the adhesive article is in a stressed state.

Methods for preparing these adhesive articles are also disclosed. In some embodiments, the method comprises providing a substrate layer with a first major surface and a second major surface, modifying the substrate layer by cutting a plurality of cuts into the substrate layer without removing material from the substrate layer, such that in an unstressed state the cuts are gaps, but the gaps are not visible to the naked eye but in a stressed state at least some of the gaps become visible to the naked eye, providing an adhesive, and forming a continuous layer of adhesive on the first major surface of the substrate layer. In some embodiments, the adhesive layer is formed on the substrate layer prior to the modification of the substrate layer.

Also disclosed are medical constructions. In some embodiments, the medical constructions comprise a surface comprising mammalian skin, and an adhesive article attached to the surface comprising mammalian skin, where the adhesive articles are described above. The adhesive article may further include a medical device.

### Brief Description of the Drawings

The present application may be more completely understood in consideration of the following detailed description of various embodiments of the disclosure in connection with the accompanying drawings.
Figure 1A shows top view photographs of articles that are embodiments of the current disclosure in an unstressed state and in two stressed states as described in Example 1.
Figure 1B shows top view photographs of articles that are comparatives to the current disclosure in an unstressed state and in two stressed states as described in Comparative Example C1.
Figure 1C shows top view photographs of articles that are comparatives to the current disclosure in an unstressed state and in two stressed states as described in Comparative Example C2.
Figure 2A shows histogram data of the articles of Figure 1A as described in Example 1.
Figure 2B shows histogram data of the articles of Figure 1B as described in Comparative Example C1.
Figure 2C shows histogram data of the articles of Figure 1C as described in Comparative Example C2.

In the following description of the illustrated embodiments, reference is made to the accompanying drawings, in which is shown by way of illustration, various embodiments in which the disclosure may be practiced. It is to be understood that the embodiments may be utilized and structural changes may be made without departing from the scope of the present disclosure. The figures are not necessarily to scale. Like numbers used in the figures refer to like components. However, it will be understood that the use of a number to refer to a component in a given figure is not intended to limit the component in another figure labeled with the same number.

### Detailed Description

The use of adhesive products in the medical industry has long been prevalent and is increasing. However, while adhesives and adhesive articles have shown themselves to be very useful for medical applications, there are also issues in the use of adhesives and adhesive articles. While many medical adhesive articles are directly applied to wound areas, a wide range of medical articles, such as tapes and drapes, are not applied to the wound area itself but rather play a supporting role to treatment such as holding absorbent materials or medical devices in place on the skin. Examples of medical devices that are held in place with tapes include tubing, catheters, ostomy appliances, sensors, and the like.

Medical adhesives have a wide array of desired properties. Among these properties are the typical adhesive requisites of sufficient peel adhesion and shear holding power, as well as flexibility so as to bend with the body, a high moisture vapor transmission rate (MVTR) and low medical adhesive-related skin injury (MARSI).

MVTR is a measure of the passage of water vapor through a substance or barrier. Because perspiration naturally occurs on the skin, if the MVTR of a material or adhesive system is low, this can result in moisture accumulation between the skin and the adhesive that can cause the adhesive to "float off" or peel away and also can promote other detrimental effects such as bacterial growth and skin irritation. Therefore, much work has focused upon the development of adhesive systems that have a high MVTR.

Medical adhesive-related skin injury (MARSI) has a significant negative impact on patient safety. Skin injury related to medical adhesive usage is a prevalent but under recognized complication that occurs across all care settings and among all age groups. In addition, treating skin damage is costly in terms of service provision, time, and additional treatments and supplies.

Skin injury occurs when the superficial layers of the skin are removed along with the medical adhesive product, which not only affects skin integrity but can cause pain and the risk of infection, increase wound size, and delay healing, all of which reduce patients' quality of life.

Medical adhesive tape can be simply defined as a pressure sensitive adhesive and a backing that acts as a carrier for the adhesive. The US Food and Drug Administration more specifically defines a medical adhesive tape or adhesive bandage as "a device intended for medical purposes that consists of a strip of fabric material or plastic, coated on one side with an adhesive, and may include a pad of surgical dressing without a disinfectant. The device is used to cover and protect wounds, to hold together the skin edges of a wound, to support an injured part of the body, or to secure objects to the skin."

While the pathophysiology of MARSI is only partially understood. Skin injury results when the skin to adhesive attachment is stronger than skin cell to skin cell attachment. When adhesive strength exceeds the strength of skin cell to skin cell interactions, cohesive failure occurs within the skin cell layer.

Current medical adhesive systems have difficulty remaining on the skin for extended periods of time because they do not address the differences in mechanical properties between the skin and the adhesive, i.e., stress/strain differentials that exist between skin and the adhesive systems. Skin typically has a low stress strain relationship that may be approximated as 0.05 MPa for strains of 1.0 or 0.02 MPa for strains of 0.4. The skin is viscoelastic and current adhesive systems are typically highly elastic. Because of the mechanical mismatch between the skin and current adhesive systems, when current adhesive systems are in place on skin and the skin moves (stretches/tension and cornpresses/compression), these adhesive systems do not move to the same extent as the skin and therefore, experience stress/strain mismatch between the adhesive system material and the skin. This mismatch results in high shear forces at the interface between the adhesive system adhesive layer and the skin upon which it is adhered. As a result of these shear forces, current adhesive systems experience edge peel, which eventually leads to peel off of the entire adhesive system.

Thus, adhesive systems are desirably designed to (1) address the mismatch of mechanical properties that exist between skin and the adhesive systems and (2) have a high MVTR. Prior adhesive systems have attempted to address the issue of mismatch of mechanical properties and the resulting edge peel, by using aggressive adhesives, i.e., adhesives that have high adhesion to skin. An adhesive's aggressiveness is defined by its initial bond strength and its sustained bond strength. However, these aggressive adhesives do not address the main problem of strain mismatch and the high shear forces that result between the skin and the adhesive. Therefore, these attempts result in systems that do not expand and contract to the same extent as the skin and remain strongly attached to the skin resulting to very high shear forces leading to pain to the wearer, and which will eventually lead to edge peel and peel off. Additionally, using an aggressive adhesive is very difficult and painful to remove from the skin when a wearer desires to remove the adhesive system. However, an adhesive that is not sufficiently aggressive, will not maintain attachment to the skin as the skin expands and contracts and will result in edge peel and peel off.

A recent attempt to solve the problem of mismatch between the skin and the adhesive article is described in PCT Publication WO 2018/125739. This publication describes an adhesive system having a first layer with a first layer material with a top and a bottom having a bottom perimeter, and a first layer adhesive on the bottom for attaching to skin, where the first layer has an inherent modulus of elasticity. The adhesive system also includes a second adhesive along only the bottom perimeter, where the first layer includes a plurality of modifications that result in the first layer having an effective modulus of elasticity that is lower than the first layer's inherent modulus of elasticity. The plurality of modifications in the first layer are perforations.

In this disclosure, adhesive articles are described that do not involve utilizing modifications of an adhesive layer or the use of multiple adhesive layers and do not involve perforating the backing layer of the adhesive article. Rather the adhesive articles of the current disclosure comprise a continuous layer of adhesive with a first major surface and a second major surface, and a substrate layer with a first major surface and the second major surface, where the first major surface of the substrate layer is in contact with the second major surface of the adhesive layer. The substrate layer comprises a plurality of cuts, where the plurality of cuts is arrayed in a pattern. In some embodiments, the pattern may be random, in other embodiments the pattern may be arrayed along at least one axis. The cuts are not perforations because they are not formed by removing material from the substrate layer, but rather are cuts that are gaps, but the gaps are not visible to the naked eye when the adhesive article is in an unstressed state, but when the adhesive article is in a stressed state at least some of the gaps are visible to the naked eye.

The use of cuts rather than perforations has a variety of advantages. Among the advantages is that the cuts are able to reduce the effective modulus without removing material from the substrate layer. The array of cuts also provides improved MVTR. An advantage of the use of cuts instead of perforations is that the array of cuts form gaps that provide a visual indicator of the application of stress to the adhesive article. As mentioned above, the cuts are gaps that are not visible to the naked eye when the adhesive article is in an unstressed state, but upon the application of stress at least some of the gaps become visible to the naked eye. In this way, the substrate itself is able to indicate when stress is applied to the adhesive article. Thus, the cuts not only provide a method for mitigating the effects of stress on the adhesive article but also provide a method for visibly detecting when stress is applied to the adhesive article. Methods for preparing these adhesive articles are also disclosed.

Also disclosed herein are medical constructions. The medical constructions comprise a surface comprising mammalian skin and an adhesive article attached to the surface comprising mammalian skin. The adhesive articles are described above and comprise a continuous layer of adhesive with a first major surface and a second major surface, and a substrate layer with a first major surface and the second major surface, where the first major surface of the substrate layer is in contact with the second major surface of the adhesive layer. The substrate layer comprises a plurality of cuts, where the plurality of cuts is arrayed in a pattern, in some embodiments the pattern is random, and in other embodiments the pattern is arrayed along at least one axis. The adhesive articles may also further include sensors or other devices that are adhered to the second major surface of the substrate layer.

Unless otherwise indicated, all numbers expressing feature sizes, amounts, and physical properties used in the specification and claims are to be understood as being modified in all instances by the term "about." Accordingly, unless indicated to the contrary, the numerical parameters set forth in the foregoing specification and attached claims are approximations that can vary depending upon the desired properties sought to be obtained by those skilled in the art utilizing the teachings disclosed herein. The recitation of numerical ranges by endpoints includes all numbers subsumed within that range (e.g. 1 to 5 includes 1, 1.5, 2, 2.75, 3, 3.80, 4, and 5) and any range within that range.

As used in this specification and the appended claims, the singular forms "a", "an", and "the" encompass embodiments having plural referents, unless the content clearly dictates otherwise. For example, reference to "a layer" encompasses embodiments having one, two or more layers. As used in this specification and the appended claims, the term "or" is generally employed in its sense including "and/or" unless the content clearly dictates otherwise.

The term "adhesive" as used herein refers to polymeric compositions useful to adhere together two adherends. Examples of adhesives are pressure sensitive adhesives and gel adhesives.

Pressure sensitive adhesive compositions are well known to those of ordinary skill in the art to possess properties including the following: (1) aggressive and permanent tack, (2) adherence with no more than finger pressure, (3) sufficient ability to hold onto an adherend, and (4) sufficient cohesive strength to be cleanly removable from the adherend. Materials that have been found to function well as pressure sensitive adhesives are polymers designed and formulated to exhibit the requisite viscoelastic properties resulting in a desired balance of tack, peel adhesion, and shear holding power. Obtaining the proper balance of properties is not a simple process.

As used herein, the term "gel adhesive" refers to a tacky semi-solid crosslinked matrix containing a liquid or a fluid that is capable of adhering to one or more substrates. The gel adhesives may have some properties in common with pressure sensitive adhesives, but they are not pressure sensitive adhesives. "Hydrogel adhesives" are gel adhesives that have water as the fluid contained within the crosslinked matrix.

The term "cut" as used herein refers to a narrow opening made in a substrate by the penetration of a cutting tool such that no material is removed from the substrate by the process of cutting. The terms "cuts" and "slits" are used interchangeably.

The term "(meth)acrylate" refers to monomeric acrylic or methacrylic esters of alcohols. Acrylate and methacrylate monomers or oligomers are referred to collectively herein as "(meth)acrylates". Materials referred to as "(meth)acrylate functional" are materials that contain one or more (meth)acrylate groups.

The terms "siloxane-based" as used herein refer to polymers or units of polymers that contain siloxane units. The terms silicone or siloxane are used interchangeably and refer to units with dialkyl or diaryl siloxane (-SiR₂O-) repeating units.

The terms "room temperature" and "ambient temperature" are used interchangeably to mean temperatures in the range of 20°C to 25°C.

The terms "Tg" and "glass transition temperature" are used interchangeably. If measured, Tg values are determined by Differential Scanning Calorimetry (DSC) at a scan rate of 10°C/minute, unless otherwise indicated. Typically, Tg values for copolymers are not measured but are calculated using the well-known Fox Equation, using the homopolymer Tg values provided by the monomer supplier, as is understood by one of skill in the art.

The term "adjacent" as used herein when referring to two layers means that the two layers are in proximity with one another with no intervening open space between them. They may be in direct contact with one another (e.g. laminated together) or there may be intervening layers.

The term "pattern" as used herein refers to a plurality of cuts, where the plurlity of cuts forms an array, and the array is in a pattern, where the pattern may be a "random pattern", meaning that there is no readily discernible repeating unit in the array, or the array may be aligned along at least one axis. In some embodiments, the array is aligned along one axis, in other embodiments, the array is aligned along more than one axis.

The terms "polymer" and "macromolecule" are used herein consistent with their common usage in chemistry. Polymers and macromolecules are composed of many repeated subunits. As used herein, the term "macromolecule" is used to describe a group attached to a monomer that has multiple repeating units. The term "polymer" is used to describe the resultant material formed from a polymerization reaction.

The term "alkyl" refers to a monovalent group that is a radical of an alkane, which is a saturated hydrocarbon. The alkyl can be linear, branched, cyclic, or combinations thereof and typically has 1 to 20 carbon atoms. In some embodiments, the alkyl group contains 1 to 18, 1 to 12, 1 to 10, 1 to 8, 1 to 6, or 1 to 4 carbon atoms. Examples of alkyl groups include, but are not limited to, methyl, ethyl, n-propyl, isopropyl, n-butyl, isobutyl, tert-butyl, n-pentyl, n-hexyl, cyclohexyl, n-heptyl, n-octyl, and ethylhexyl.

The term "aryl" refers to a monovalent group that is aromatic and carbocyclic. The aryl can have one to five rings that are connected to or fused to the aromatic ring. The other ring structures can be aromatic, non-aromatic, or combinations thereof. Examples of aryl groups include, but are not limited to, phenyl, biphenyl, terphenyl, anthryl, naphthyl, acenaphthyl, anthraquinonyl, phenanthryl, anthracenyl, pyrenyl, perylenyl, and fluorenyl.

The term "alkylene" refers to a divalent group that is a radical of an alkane. The alkylene can be straight-chained, branched, cyclic, or combinations thereof. The alkylene often has 1 to 20 carbon atoms. In some embodiments, the alkylene contains 1 to 18, 1 to 12, 1 to 10, 1 to 8, 1 to 6, or 1 to 4 carbon atoms. The radical centers of the alkylene can be on the same carbon atom (i.e., an alkylidene) or on different carbon atoms.

The terms "free radically polymerizable" and "ethylenically unsaturated" are used interchangeably and refer to a reactive group which contains a carbon-carbon double bond which is able to be polymerized via a free radical polymerization mechanism.

The term "gap" as used herein refers to a void space in a substrate that passes through the entire thickness of the substrate. Gaps can be prepared by cutting, slitting, boring, etc. In the current disclosure, gaps are described as not visible to the naked eye when the substrate is in an unstressed state, and the gaps are described as being visible to the naked eye and as forming "apertures" when in a stressed state. The term "aperture" as used herein is used according to the typical definition and is a space through which light passes. The term "hole" as used herein refers to a void space in the surface of a substrate that is visible to the naked eye in an unstressed state and a stressed state, being an aperture under both an unstressed state and a stressed state.

Disclosed herein are adhesive articles suitable for a wide range of medical uses. In some embodiments, the adhesive articles comprise a continuous layer of adhesive with a first major surface and a second major surface, and a substrate layer with a first major surface and the second major surface, where the first major surface of the substrate layer is in contact with the second major surface of the adhesive layer. The substrate layer comprises a plurality of cuts, wherein the plurality of cuts is arrayed in a pattern. In some embodiments, the pattern may be random, in other embodiments the pattern may be arrayed along at least one axis. The cuts are gaps, but these gaps are not visible to the naked eye when the adhesive article is in an unstressed state, but at least some of the gaps become visible to the naked eye when the adhesive article is in a stressed state.

A wide array of adhesives is suitable for use in the adhesive layer of the adhesive articles of this disclosure. In some embodiments, the adhesive comprises a pressure sensitive adhesive, in other embodiments the adhesive comprises a gel adhesive. Suitable pressure sensitive adhesives include (meth)acrylate-based pressure sensitive adhesives and siloxane-based pressure sensitive adhesives.

One suitable class of (meth)acrylate-based pressure sensitive adhesives include copolymers derived from: (A) at least one monoethylenically unsaturated alkyl (meth) acrylate monomer (i.e., alkyl acrylate and alkyl methacrylate monomer); and (B) at least one monoethylenically unsaturated free-radically copolymerizable reinforcing monomer. The reinforcing monomer has a homopolymer glass transition temperature (Tg) higher than that of the alkyl (meth)acrylate monomer and is one that increases the glass transition temperature and cohesive strength of the resultant copolymer. Herein, "copolymer" refers to polymers containing two or more different monomers, including terpolymers, tetrapolymers, etc.

Monomer A, which is a monoethylenically unsaturated alkyl acrylate or methacrylate (i.e., (meth)acrylic acid ester), contributes to the flexibility and tack of the copolymer. Generally, monomer A has a homopolymer Tg of no greater than about 0°C. Typically, the alkyl group of the (meth)acrylate has an average of about 4 to about 20 carbon atoms, or an average of about 4 to about 14 carbon atoms. The alkyl group can optionally contain oxygen atoms in the chain thereby forming ethers or alkoxy ethers, for example. Examples of monomer A include, but are not limited to, 2-methylbutyl acrylate, isooctyl acrylate, lauryl acrylate, 4- methyl-2-pentyl acrylate, isoamyl acrylate, sec-butyl acrylate, n-butyl acrylate, n-hexyl acrylate, 2-ethylhexyl acrylate, n-octyl acrylate, n-decyl acrylate, isodecyl acrylate, isodecyl methacrylate, and isononyl acrylate. Other examples include, but are not limited to, poly-ethoxylated or -propoxylated methoxy (meth)acrylates such as acrylates of CARBOWAX (commercially available from Union Carbide) and NK ester AM90G (commercially available from Shin Nakamura Chemical, Ltd., Japan). Suitable monoethylenically unsaturated (meth)acrylates that can be used as monomer A include isooctyl acrylate, 2-ethyl-hexyl acrylate, and n- butyl acrylate. Combinations of various monomers categorized as an A monomer can be used to make the copolymer.

Monomer B, which is a monoethylenically unsaturated free- radically copolymerizable reinforcing monomer, increases the glass transition temperature and cohesive strength of the copolymer. Generally, monomer B has a homopolymer Tg of at least about 10°C. Typically, monomer B is a reinforcing (meth)acrylic monomer, including an acrylic acid, a methacrylic acid, an acrylamide, or a (meth)acrylate. Examples of monomer B include, but are not limited to, acrylamides, such as acrylamide, methacrylamide, N-methyl acrylamide, N-ethyl acrylamide, N- hydroxyethyl acrylamide, diacetone acrylamide, N,N-dimethyl acrylamide, N, N-diethyl acrylamide, N-ethyl-N-aminoethyl acrylamide, N-ethyl-N- hydroxyethyl acrylamide, N,N-dihydroxyethyl acrylamide, t-butyl acrylamide, N,N-dimethylaminoethyl acrylamide, and N-octyl acrylamide. Other examples of monomer B include itaconic acid, crotonic acid, maleic acid, fumaric acid, 2,2-(diethoxy)ethyl acrylate, 2-hydroxyethyl acrylate or methacrylate, 3-hydroxypropyl acrylate or methacrylate, methyl methacrylate, isobornyl acrylate, 2-(phenoxy)ethyl acrylate or methacrylate, biphenylyl acrylate, t-butylphenyl acrylate, cyclohexyl acrylate, dimethyladamantyl acrylate, 2-naphthyl acrylate, phenyl acrylate, N-vinyl formamide, N-vinyl acetamide, N-vinyl pyrrolidone, and N-vinyl caprolactam. Particularly suitable reinforcing acrylic monomers that can be used as monomer B include acrylic acid and acrylamide. Combinations of various reinforcing monoethylenically unsaturated monomers categorized as a B monomer can be used to make the copolymer.

Generally, the (meth)acrylate copolymer is formulated to have a resultant Tg of less than about 0°C and more typically, less than about -10°C. Such (meth)acrylate copolymers generally include about 60 parts to about 98 parts per hundred of at least one monomer A and about 2 parts to about 40 parts per hundred of at least one monomer B. In some embodiments, the (meth)acrylate copolymers have about 85 parts to about 98 parts per hundred or at least one monomer A and about 2 parts to about 15 parts of at least one monomer B.

Examples of suitable (meth)acrylate-based pressure sensitive adhesives that can be applied to skin are described in U.S. Patent No. RE 24,906. In some embodiments, a 97:3 iso-octyl acrylate:acrylamide copolymer adhesive can be used or a 70:15:15 isooctyl acrylate: ethyleneoxide acrylate:acrylic acid terpolymer, as described in US Patent No. 4,737,410. Other useful adhesives are described in US Patent Nos. 3,389,827, 4,112,213, 4,310,509, and 4,323,557.

Another class of suitable pressure sensitive adhesive is siloxane-based adhesives. Siloxane-based pressure sensitive adhesives include, for example, those described in US Patent Nos. 5,527,578 and 5,858,545; and PCT Publication No. WO 00/02966. Specific examples include polydiorganosiloxane polyurea copolymers and blends thereof, such as those described in U.S. Patent No. 6,007,914, and polysiloxane-polyalkylene block copolymers. Other examples of siloxane pressure sensitive adhesives include those formed from silanols, silicone hydrides, siloxanes, epoxides, and (meth)acrylates. When the siloxane pressure sensitive adhesive is prepared from (meth)acrylate-functional siloxanes, the adhesive is sometimes referred to as a siloxane (meth)acrylate.

The siloxane-based adhesive compositions comprise at least one siloxane elastomeric polymer and may contain other components such as tackifying resins. The elastomeric polymers include for example, urea-based siloxane copolymers, oxamide-based siloxane copolymers, amide-based siloxane copolymers, urethane-based siloxane copolymers, and mixtures thereof.

Useful siloxane polyurea block copolymers are disclosed in, e.g., U.S. Patent Nos. 5,512,650, 5,214,119, 5,461,134, and 7,153,924 and PCT Publication Nos. WO 96/35458, WO 98/17726, WO 96/34028, WO 96/34030 and WO 97/40103.

Another useful class of siloxane elastomeric polymers are oxamide-based polymers such as polydiorganosiloxane polyoxamide block copolymers. Examples of polydiorganosiloxane polyoxamide block copolymers are presented, for example, in US Patent Publication No. 2007-0148475.

Another useful class of siloxane elastomeric polymer is amide-based siloxane polymers. Such polymers are similar to the urea-based polymers, containing amide linkages (-N(D)-C(O)-) instead of urea linkages (-N(D)-C(O)-N(D)-), where C(O) represents a carbonyl group and D is a hydrogen or alkyl group.

Another useful class of siloxane elastomeric polymer is urethane-based siloxane polymers such as siloxane polyurea-urethane block copolymers. Siloxane polyurea-urethane block copolymers include the reaction product of a polydiorganosiloxane diamine (also referred to as siloxane diamine), a diisocyanate, and an organic polyol. Such materials are structurally very similar to the structure of Formula I except that the - N(D)-B-N(D)- links are replaced by -O-B-O- links. Examples are such polymers are presented, for example, in US Patent No. 5,214,119.

In some embodiments, the siloxane-based pressure sensitive adhesive further comprises a siloxane tackifying resin. Siloxane tackifying resins have in the past been referred to as "silicate" tackifying resins, but that nomenclature has been replaced with the term "siloxane tackifying resin". The siloxane tackifying resins are added in sufficient quantity to achieve the desired tackiness and level of adhesion. In some embodiments, a plurality of siloxane tackifying resins can be used to achieve desired performance.

Suitable siloxane tackifying resins are commercially available from sources such as Dow Corning (e.g., DC 2-7066), Momentive Performance Materials (e.g., SR545 and SR1000), and Wacker Chemie AG (e.g., BELSIL TMS-803).

Another class of adhesives used in medical applications are silicone gels. As used herein, the terms "siloxane" and "silicone" are used interchangeably. The term siloxane is replacing silicone in common usage, but both terms are used in the art. Silicone gel (crosslinked poly dimethylsiloxane ("PDMS")) materials have been used for dielectric fillers, vibration dampers, and medical therapies for promoting scar tissue healing. Lightly crosslinked silicone gels are soft, tacky, elastic materials that comprise relatively high levels of fluids (liquids). Silicone gels are typically softer than silicone pressure sensitive adhesives, resulting in less discomfort when adhered to skin. The combination of reasonable adhesive holding power on skin and low skin trauma upon removal, make silicone gels suitable for gentle to skin adhesive applications.

Silicone gel adhesives provide good adhesion to skin with gentle removal force and have the ability to repositioned. Examples of commercially available silicone gel adhesive systems include products marketed with the trade names: Dow Corning MG 7-9850, WACKER 2130, BLUESTAR 4317 and 4320, and NUSIL 6345 and 6350. These gentle to the skin adhesives are formed by an addition cure reaction between vinyl-terminated PDMS and hydrogen terminated PDMS, in the presence of a hydrosilylation catalyst (e.g., platinum complex). Vinyl-terminated and hydrogen terminated PDMS chains are referred to as 'functionalized' silicones due to their specific chemical moieties. Individually, such functional silicones are generally not reactive; however, together they form a reactive silicone system. Additionally, silicone resins (tackifiers sometimes referred to as "silicate resins") and PDMS with multiple hydrogen functionalities (crosslinkers) can be formulated to modify the adhesive properties of the gel.

The substrate layer is typically a thin-film material. The film material is rigid enough to provide support to the adhesive article. The substrate layer is modified by cutting to make it flexible enough that it is conformable to anatomical surfaces. As such, when applied to an anatomical surface, it conforms to the surface even when the surface is moved and can stretch and retract. The modification process of the current disclosure permits a wide range of material films to be used that otherwise would not be conformable to anatamical surfaces. Among the classes of materials suitable are thermoplastics, elatomers, and nonwovens. The substrate layer may be prepared from a wide range of materials including polyolefins, polyurethanes, polyestesr, polyether block amides, or natural fiber-based materials. One particularly suitable substrate is the SONTARA nonwoven material from Sontara America, which is a tightly enmeshed nonwoven film with no holes suitable for use in medical barrier and drape applications.

The substrate layer has a wide range of thicknesses. In some embodiments, the thickness is at least 10 micrometers, up to 254 micrometers (10 mils), and in some embodiments the thickness will be from 25 micrometers (1 mil) up to 178 micrometers (7 mils) thick. A wide range of intermediate thicknesses are also suitable.

As mentioned above, the substrate layer contains a plurality of cuts arrayed in a pattern, in some embodiments the pattern may be random, in other embodiments, the pattern is arrayed along at least one axis. The two-dimensional surface of the substrate layer can be viewed as having two primary orthogonal axes, frequently referred to as an x axis and a y axis. Because of how films are made, often the x axis is described as the "Machine Direction" or MD and the orthogonal y axis is described as the "Cross Direction" or CD. Typically, the plurality of cuts is arrayed in a pattern along the Machine Direction. By this it is meant that the lengths of the plurality of cuts are aligned along the Machine Direction of the substrate layer.

The plurality of cuts arrayed in a pattern are gaps, but these gaps are essentially not visible to the naked eye when the substrate layer is in an unstressed state. At least some of the gaps become visible to the naked eye when the adhesive article is in a stressed state. As used herein the term "stressed state" comprises stretching, bending or a combination thereof. Because the cuts are very thin (i.e. have essentially no width) in the unstressed state, they are typically described by their length and a depth. Generally, the cut extends through the full thickness of the substrate layer, so the depth is the same as the thickness of the substrate layer. One parameter that can be descriptive of the relationship of the length to the width of the cut is the aspect ratio. The term "aspect ratio" is typically used to describe particles, but as used herein it is used to describe hole size or void regions where material is not present. In some embodiments, the aspect ratio (the ratio of length to width) for the cuts is greater than 1000. Upon application of a stress, the aspect ratio for the cuts decreases. An example of a suitable cut is one that is 1 centimeter long and 5 micrometers wide.

The cuts may be more complex than the simple linear shape described above. The cuts may have a variety of two-dimensional shapes such as crosses, asterisks, chevrons, letters, numbers, and the like as long as the shape is not visible to the naked eye in the unstressed state and at least some of the cuts become gaps that are visible to the naked eye when the adhesive article is in a stressed state. Additionally, not only do the gaps become visible to the naked eye upon application of stress to the article, but also the gaps become apertures that light can pass through. In this way, upon application of stress, the formed apertures permit viewing through the substrate layer.

In some embodiments, the cuts are arrayed in a pattern along the Machine Direction of the substrate. Often the cuts are formed by a process known as "skip slitting". In this process, a series of discontinuous slits are formed in the substrate in a linear manner. If one were to follow a line of slits along the substrate surface one would encounter a slit, the slit would end and there would be a region that is not slit, then a new slit would be encountered, that slit would end, a region that is not slit would be encountered, and so forth. This type of pattern is described in PCT Publication No. WO 19/043621. In these patterns, the region between the end of one slit and the start of the next slit is called a bridging region. While a wide array of patterns is suitable, in some embodiments, the patterns have a 50% offset. By this it is meant that when two linear arrays are present in the Machine Direction, when observed in the Cross Direction, the bridging region of the first array corresponds to slits in the second array and vice versa.

In some embodiments, the plurality of cuts is arrayed in a pattern along more than one axis. By this it is meant that at least some of cuts have lengths that are not aligned with the Machine Direction of the substrate layer but are offset from alignment by an angle of up to 90°. Lengths that are offset from alignment with the MD of the substrate layer by 90° are aligned in the Cross Direction.

The plurality of cuts can be made in a number of different ways as long as the method does not involve removing material from the substrate layer and the cuts form gaps that are essentially not visible to the naked eye in the unstressed state and at least some of gaps become visible to the naked eye when the adhesive article is in a stressed state. Among the methods are those in which the cuts are introduced into the substrate layer when the layer is formed for example by extrusion, molding, machining and the like. Other methods are those in which the cuts are introduced into the substrate layer after the substrate layer is formed such as by cutting using a cutting tool such as a knife, a linear blade, a rotary die blade, a water jet, or a laser beam, or by stamping using a stamping tool. In some embodiments, the cuts are made by feeding the substrate layer into a nip containing a rotary die blade and an anvil such that the die cuts through the substrate layer to form the cut pattern.

In some embodiments, the substrate layer is not modified until the adhesive layer is applied to it to form an article and then the cuts are formed in the substrate layer. As will be described below, in some embodiments that cuts are only present in the substrate layer. In other embodiments, the cuts are present in the substrate layer and the adhesive layer.

The substrate layer of the present articles has a lower effective modulus of elasticity in at least one axis than an identical substrate layer without the plurality of cuts. In other words, modifying the substrate layer with a plurality of cuts reduces the effective modulus of elasticity. The elastic modulus (also known as the modulus of elasticity) is a quantity that measures an object or substance's resistance to being deformed elastically (i.e., non-permanently) when a stress is applied to it. The elastic modulus of an object is defined as the slope of its stress-strain curve in the elastic deformation region. Effective modulus of elasticity is defined in the art as the ratios of the average stresses to the average strains that result in the body when it is subject to pure shear or pure compression on its outer boundary. In the current disclosure, the effective elastic modulus is the elastic modulus of the substrate layer or of an adhesive article that contains the substrate layer after the substrate layer has been modified by cutting.

While it might seem that perforations of the substrate layer would be an equally suitable method for reducing the effective modulus of elasticity, it has been found that the use of perforations does not provide the same reduction in the modulus of elasticity. In this context, the term "perforations" is used according to its commonly used definition and refers to a hole through a substrate prepared by a method that removes material from the substrate. Unlike the cuts of the current disclosure, perforations are readily visible to the naked eye whether in an unstressed or a stressed state.

The adhesive articles of the present disclosure have desirable optical properties. Among the desirable optical properties is the property that when the adhesive article is in a stressed state the adhesive article has optical properties different from the optical properties of the adhesive article in the unstressed state. The difference in optical properties are related to property of the adhesive article that the plurality of cuts arrayed in a pattern are gaps that are not visible to the naked eye when the substrate layer is in an unstressed state, and at least some of the gaps become visible to the naked eye when the adhesive article is in a stressed state. For example, the gaps becoming visible in the substrate layer by application of stress is evidenced by portions of the substrate layer changing from being opaque to being light transmitting. In this way, the surface to which the stressed adhesive article is applied can become visible. If the cuts are not also present in the adhesive layer, applying stress to adhesive article can cause portions of the adhesive layer to become visible.

The detection of changes in the optical properties are typically detectable by the naked eye. As mentioned above, the cuts in the substrate layer are not visible to the naked eye but application of stress to the adhesive article causes the cuts to become visible to the naked eye.

The changes in optical properties upon application of a stress to the adhesive article are useful for a variety of reasons. For example, application of a stress to the adhesive article when applied to a patient can be generated by swelling due to injury or from exudate produced by a wound. In some embodiments, a visible indication of stress be applied to the adhesive articles can result from simple motion, bending, twisting, etc. by the patient wearing the adhesive article, and thus indicates that the motion, bending, twisting, etc. is excessive.

Also disclosed herein are adhesive articles that not only comprise the adhesive layer and substrate layer but also further comprise a device, where the device is attached to the second major surface of the substrate layer. A wide range of devices are suitable, such as sensors or monitors. An advantage of the adhesive articles of this disclosure is that the flexibility permits the adhesive article to be worn for extended periods of time without the issues described above. Thus, the adhesive articles of this disclosure can be worn for extended periods such as days, weeks or even longer. This can be particularly important with adhesive articles that include devices that are meant to be worn by a patient for extended periods of time. An additional advantage of adhesive articles that include the modified substrates of this disclosure, is that the reduced effective modulus of elasticity provides a flexible, dampening layer between the skin which is flexible and a device which is generally rigid. This flexible dampening layer permits increased movement by the wearer of the device without causing stress on the device or connections between the device and the skin.

Also disclosed herein are medical constructions. The medical constructions comprise a surface comprising mammalian skin, and an adhesive article such as described above attached to the surface comprising mammalian skin. These adhesive articles comprise a continuous layer of adhesive with a first major surface and a second major surface, and a substrate layer with a first major surface and the second major surface, where the first major surface of the substrate layer is in contact with the second major surface of the adhesive layer. The substrate layer comprises a plurality of cuts, wherein the plurality of cuts is arrayed in a pattern, in some embodiments the pattern is random, in other embodiments, the pattern is arrayed along at least one axis, and the cuts are gaps that are not visible to the naked eye when the adhesive article is in an unstressed state, and at least some of the gaps become visible to the naked eye when the adhesive article is in a stressed state. The first major surface of the adhesive layer is in contact with and adhesively bonded to the surface comprising mammalian skin. As described above, the adhesive article may further comprise a device, where the device is attached to the second major surface of the substrate layer.

Also disclosed herein are methods of preparing adhesive articles. In some embodiments, the method comprises providing a substrate layer with a first major surface and a second major surface, modifying the substrate layer by cutting a plurality of cuts into the substrate layer, such that in an unstressed state the cuts are gaps that are not visible to the naked eye and in a stressed state at least some of the gaps become visible to the naked eye, providing an adhesive, and forming a continuous layer of adhesive on the first major surface of the substrate layer.

In some embodiments, modifying the substrate layer comprising cutting a plurality of cuts into the substrate layer with a knife, a blade, a water jet, or a laser beam. A wide variety of techniques are suitable for modifying the substrate layer. Examples of modification include cutting using a cutting tool such as a knife, a linear blade, a rotary die blade, a water jet, or a laser beam, or by stamping using a stamping tool. In some embodiments, the cuts are made by feeding the substrate layer into a nip containing a rotary die blade and an anvil such that the die cuts through the substrate layer to form the cut pattern.

In many embodiments, the substrate layer is modified prior to forming a continuous layer of adhesive on the first major surface of the substrate layer. In this way, the substrate layer can be modified in a different location or at a different time than the formation of the adhesive article. In some embodiments, the continuous layer of adhesive on the first major surface of the substrate layer is formed prior to modifying the substrate layer. The modification of the substrate layer may involve just modification of the substrate layer or the modification of the substrate layer may involve modification of both the substrate layer and the adhesive layer. In these embodiments, the plurality of cuts present in the substrate layer are also present in the adhesive layer.

As described above, the method of forming an adhesive article further comprises attachment of a device to the second major surface of the substrate layer. Suitable devices are described above.

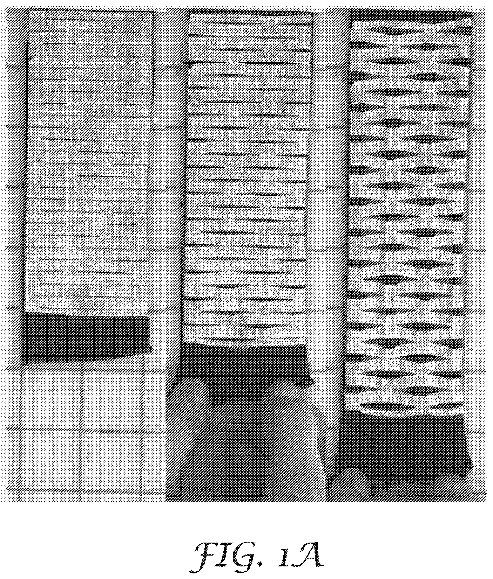

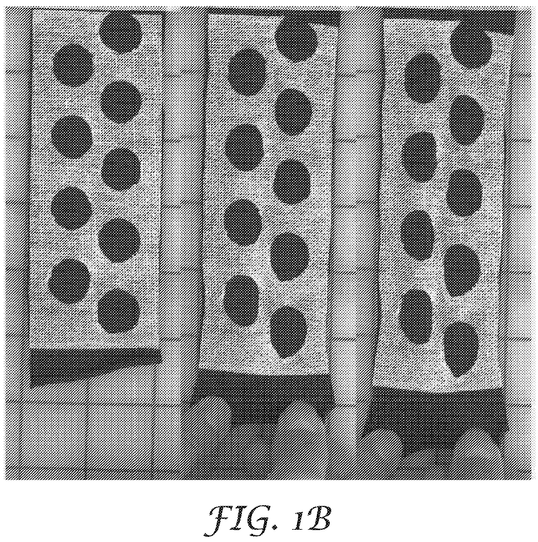

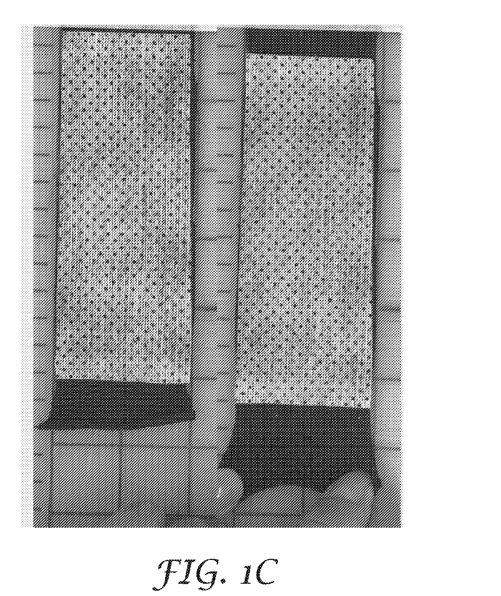

The articles of this disclosure may be more fully understood by the figures, which are more fully explained in the Examples section below. Figure 1A shows an article of this disclosure where the gaps formed by an array of cuts are essentially not visible in the unstressed state and upon application of stress to the article by pulling the underlying substrate on which the article is mounted, the gaps become visible and the underlying substrate can be clearly seen through the gaps. Additionally, as the underlying substrate is stretched, the article elongates, demonstrating that the modulus of elasticity that permits the article to elongate. Figure 1B and 1C show comparative substrates with perforations. As can be seen the perforations are visible both in the stressed and the unstressed states. Also, the application of stress to the underlying substrate on which the articles are mounted does not cause the perforated substrate to elongate, indicating that the modulus of elasticity of the article does not permit elongation of the article.

### Examples

These examples are merely for illustrative purposes only and are not meant to be limiting on the scope of the appended claims. The following abbreviations are used: mm = millimeters; kHz = kiloHertz.

### Test specimen preparation and measurement

A strip of Sontara nonwoven material (Sontara America, Inc., Candler, NC) was coated with a medical grade adhesive. Patterns were laser cut (laser settings were 13% power, 75 kHz, 1000 mm/s, 2 passes) in the coated Sontara material, including slits (Example 1) and round holes of two diameters (Comparative Examples C1 and C2). The patterned and coated Sontara was then laminated to spandex-type fabric with a hand roller so that unlaminated spandex fabric was exposed at the top and bottom of the samples. Test specimens were cut out with 2 inches by 5 inches (approximately 51 x 127 mm) pieces of Sontara material centered in the middle of the specimen and about 12 mm of spandex-type fabric exposed at each end. The test specimens were fastened to a measurement grid by the exposed spandex at the top and the exposed spandex-type fabric at the bottom was gripped to stretch the specimen. The specimen shapes and cutting patterns can be seen in Fig. 1A (Example 1), Fig. 1B (Comparative Example C1), and Fig. 1C (Comparative Example C2).

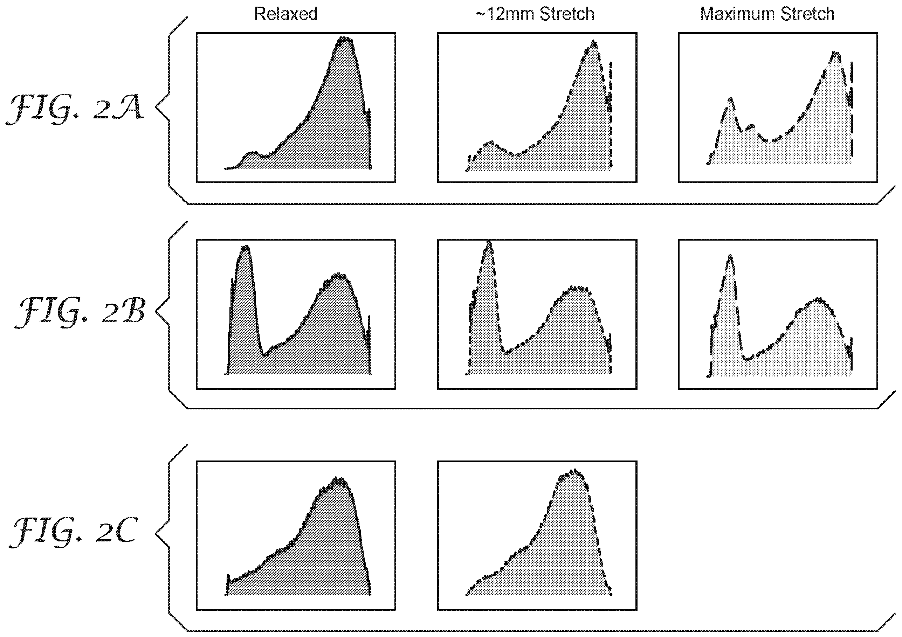

The specimens were imaged using a mounted iPhone X camera and histograms were calculated. Each specimen was then stretched approximately 12 mm, images were recorded, and histograms were calculated. Lastly, the samples were stretched to the maximum amount and the imaging and histogram calculation repeated. The images are shown in Figs. 1A-C. The histograms were calculated using ImageJ (an image processing program available at imagej.net) and are shown in Fig. 2A-C.

### Discussion of Results

The Sontara fabric resists stretching and deformation. Skip slitting allowed for a larger maximum stretch of the test specimen, and the resulting histograms changed due to the exposure of the dark, underlying spandex material through the opened slits. This is due to the ability of the skip slit pattern to enable deformation, as shown in Fig. 1A. Fig. 2A provides the histograms, respectively, of the skip slit specimen in a relaxed (unstretched) state, stretched approximately 12 mm, and maximally stretched, left to right.

The specimens with round perforations did not facilitate stretching, although the shapes deformed into ovals. This deformation did not expose significantly more spandex material, and the histograms does not show significant change. The specimen with 12 mm perforations (Fig. 2B, left, center, right) allowed for a slightly greater extension ratio but did not result in a significant difference between histograms. The specimen with 2 mm perforations also did not significantly deform into ovals (Fig. 2C, left, center).

The visible change is based on the layers of Sontara and spandex fabric having different optical responses. The optical performance is captured by the histogram comparison of the perforated/slit area in the relaxed and stretched state. The stretched slit samples showed significant changes in mean, mode, and standard deviation of the greyscale values compared to the perforated ones. The sign of the change in values is related to the top and bottom contrast choices. The magnitude of change is related pattern's ability to expose the underlying material and the amount of stretch.

## Claims

1. Adhesive articles comprising:
a continuous layer of adhesive with a first major surface and a second major surface;
and
a substrate layer with a first major surface and the second major surface, wherein the
first major surface of the substrate layer is in contact with the second major surface of the adhesive layer, and
wherein the substrate layer comprises a plurality of cuts, wherein the plurality of cuts are narrow openings made in the substrate layer by penetration of a cutting tool such that no material is removed from the substrate layer by the process of cutting,
wherein the plurality of cuts is arrayed in a pattern, and wherein the cuts are gaps prepared by cutting or slitting, but
the gaps are not visible to the naked eye and a ratio of a length to a width for the cuts is greater than 1000 when the adhesive article is in an unstressed state, and
at least some of the cuts become gaps that are visible to the naked eye, the ratio of the length to the width for the cuts decreases, and the gaps forming apertures through which light passes, when the adhesive article is in a stressed state.

2. The adhesive article of claim 1, wherein at least some of the gaps visible to the naked eye when the adhesive article is in a stressed state provide apertures through which one can view through the adhesive article.

3. The adhesive article of claim 1, wherein the plurality of cuts is arrayed in a pattern along one axis or more than one axis.

4. The adhesive article of claim 1, wherein the adhesive comprises a gel adhesive or a pressure sensitive adhesive.

5. The adhesive article of claim 1, wherein the substrate layer comprises a thermoplastic film of polyolefin, polyurethane, polyester, or polyether block amide.

6. The adhesive article of claim 1, wherein the stressed state comprises stretching, bending or a combination thereof.

7. The adhesive article of claim 1, wherein when the adhesive article is in a stressed state the adhesive article has optical properties different from the optical properties of the adhesive article in the unstressed state.

8. The adhesive article of claim 1, wherein the substrate layer has a lower effective modulus of elasticity in at least one axis than an identical substrate layer without the plurality of cuts.

9. The adhesive article of claim 1, further comprising a device, wherein the device is attached to the second major surface of the substrate layer, wherein the device comprises a monitor or a sensor.

10. A medical construction comprising:
an adhesive article as defined in any of the preceding claims attachable to a surface comprising mammalian skin, wherein the adhesive article comprises:
a continuous layer of adhesive with a first major surface and a second major surface; and
a substrate layer with a first major surface and the second major surface, wherein the first major surface of the substrate layer is in contact with the second major surface of the adhesive layer, and wherein the substrate layer comprises a plurality of cuts, wherein the plurality of cuts are narrow openings made in the substrate layer by penetration of a cutting tool such that no material is removed from the substrate layer by the process of cutting, wherein the plurality of cuts is arrayed in a pattern, and wherein the cuts are gaps prepared by cutting or slitting, but
at least some of the gaps are not visible to the naked eye and a ratio of a length to a width for the cuts is greater than 1000 when the adhesive article is in a first state, and
at least some of the gaps that are not visible to the naked eye in the first state become gaps that are visible to the naked eye, the gaps forming apertures through which light passes, and the ratio of the length to the width of the cuts decreases when the adhesive article is in a second state, wherein
the second state is a state of higher stress of bending, stetching or a combination thereof, and wherein
the first major surface of adhesive layer is configured to get in contact with the surface comprising mammalian skin.

11. A method of preparing an adhesive article as defined in any of the preceding claims comprising
providing a substrate layer with a first major surface and a second major surface;
modifying the substrate layer by cutting a plurality of cuts into the substrate layer without removing material from the substrate layer, such that
in an unstressed state, the cuts are gaps, but the gaps are not visible to the naked eye and a ratio of a length to a width for the cuts is greater than 1000, but
in a stressed state at least some of the gaps become visible to the naked eye, the ratio decreases, and the gaps form apertures through which light passes;
providing an adhesive; and
forming a continuous layer of adhesive on the first major surface of the substrate layer.

12. The method of claim 11, wherein modifying the substrate layer by cutting a plurality of cuts into the substrate layer comprises cutting with a knife, a blade, a water jet, or a laser beam.

13. The method of claim 11, wherein the continuous layer of adhesive on the first major surface of the substrate layer is formed prior to modifying the substrate layer.

14. The method of claim 13, wherein the plurality of cuts is also present in the adhesive layer.

## Patentansprüche

1. Klebeartikel, aufweisend:
eine fortlaufende Schicht von Klebstoff mit einer ersten Hauptoberfläche und einer zweiten Hauptoberfläche;
und
eine Substratschicht mit einer ersten Hauptoberfläche und der zweiten Hauptoberfläche, wobei die
erste Hauptoberfläche der Substratschicht mit der zweiten Hauptoberfläche der Klebeschicht in Kontakt ist, und
wobei die Substratschicht eine Mehrzahl von Schnitten aufweist, wobei die Mehrzahl von Schnitten schmale Öffnungen sind, die durch Eindringung eines Schneidwerkzeugs in die Substratschicht erzeugt werden, derart, dass durch den Schneidprozess kein Material aus der Substratschicht entfernt wird,
wobei die Mehrzahl von Schnitten in einem Muster angeordnet ist, und wobei die Schnitte Spalte sind, die durch Schneiden oder Schlitzen hergestellt werden, aber
die Spalte für das bloße Auge nicht sichtbar sind und ein Verhältnis einer Länge zu einer Breite für die Schnitte größer als 1000 ist, wenn der Klebeartikel in einem unbelasteten Zustand ist, und
mindestens einige der Schnitte zu Spalten, die für das bloße Auge sichtbar sind, werden, das Verhältnis der Länge zu der Breite für die Schnitte abnimmt und die Spalte Öffnungen, durch die Licht hindurchtritt, bilden, wenn der Klebeartikel in einem belasteten Zustand ist.

2. Der Klebeartikel nach Anspruch 1, wobei mindestens einige der Spalte, die für das bloße Auge sichtbar sind, wenn der Klebeartikel in einem belasteten Zustand ist, Öffnungen, durch die man durch den Klebeartikel hindurchsehen kann, bereitstellen.

3. Der Klebeartikel nach Anspruch 1, wobei die Mehrzahl von Schnitten in einem Muster entlang einer Achse oder mehr als einer Achse angeordnet ist.

4. Der Klebeartikel nach Anspruch 1, wobei der Klebstoff einen Gelklebstoff oder einen Haftklebstoff aufweist.

5. Der Klebeartikel nach Anspruch 1, wobei die Substratschicht eine thermoplastische Folie aus Polyolefin, Polyurethan, Polyester oder Polyetherblockamid aufweist.

6. Der Klebeartikel nach Anspruch 1, wobei der belastete Zustand Dehnen, Biegen oder eine Kombination davon aufweist.

7. Der Klebeartikel nach Anspruch 1, wobei, wenn der Klebeartikel in einem belasteten Zustand ist, der Klebeartikel über optische Eigenschaften, die sich von den optischen Eigenschaften des Klebeartikels in dem unbelasteten Zustand unterscheiden, verfügt.

8. Der Klebeartikel nach Anspruch 1, wobei die Substratschicht in mindestens einer Achse über einen niedrigeren effektiven Elastizitätsmodul als eine identische Substratschicht ohne die Mehrzahl von Schnitten verfügt.

9. Der Klebeartikel nach Anspruch 1, ferner aufweisend eine Vorrichtung, wobei die Vorrichtung an der zweiten Hauptoberfläche der Substratschicht angebracht ist, wobei die Vorrichtung einen Monitor oder einen Sensor aufweist.

10. Eine medizinische Konstruktion, aufweisend:
einen Klebeartikel, wie in einem der vorstehenden Ansprüche definiert, der an eine Oberfläche, aufweisend Säugetierhaut, anbringbar ist, wobei der Klebeartikel aufweist:
eine fortlaufende Schicht von Klebstoff mit einer ersten Hauptoberfläche und einer zweiten Hauptoberfläche; und
eine Substratschicht mit einer ersten Hauptoberfläche und der zweiten Hauptoberfläche, wobei die erste Hauptoberfläche der Substratschicht mit der zweiten Hauptoberfläche der Klebeschicht in Kontakt ist, und wobei die Substratschicht eine Mehrzahl von Schnitten aufweist, wobei die Mehrzahl von Schnitten schmale Öffnungen sind, die durch Eindringung eines Schneidwerkzeugs in die Substratschicht erzeugt werden, derart, dass durch den Schneidprozess kein Material aus der Substratschicht entfernt wird, wobei die Mehrzahl von Schnitten in einem Muster angeordnet ist, und wobei die Schnitte Spalte sind, die durch Schneiden oder Schlitzen hergestellt werden, aber
mindestens einige der Spalte für das bloße Auge nicht sichtbar sind und ein Verhältnis einer Länge zu einer Breite für die Schnitte größer als 1000 ist, wenn der Klebeartikel in einem ersten Zustand ist, und
mindestens einige der Spalte, die in dem ersten Zustand für das bloße Auge nicht sichtbar sind, zu Spalten, die für das bloße Auge sichtbar sind, werden, wobei die Spalte Öffnungen, durch die Licht hindurchtritt, bilden und das Verhältnis der Länge zu der Breite der Schnitte abnimmt, wenn der Klebeartikel in einem zweiten Zustand ist, wobei
der zweite Zustand ein Zustand höherer Belastung durch Biegung, Dehnung oder eine Kombination davon ist, und wobei
die erste Hauptoberfläche von Klebeschicht konfiguriert ist, um mit der Oberfläche, aufweisend Säugetierhaut, in Kontakt zu kommen.

11. Ein Verfahren zum Herstellen eines Klebeartikels wie in einem der vorstehenden Ansprüche definiert, aufweisend:
Bereitstellen einer Substratschicht mit einer ersten Hauptoberfläche und einer zweiten Hauptoberfläche;
Modifizieren der Substratschicht durch Schneiden einer Mehrzahl von Schnitten in die Substratschicht, ohne Material von der Substratschicht zu entfernen, derart, dass
in einem unbelasteten Zustand die Schnitte Spalte sind, aber die Spalte für das bloße Auge nicht sichtbar sind und ein Verhältnis einer Länge zu einer Breite für die Schnitte größer als 1000 ist, aber
in einem belasteten Zustand mindestens einige der Spalte für das bloße Auge sichtbar werden, das Verhältnis abnimmt und die Spalte Öffnungen, durch die Licht hindurchtritt, bilden;
Bereitstellen eines Klebstoffs; und
Ausbilden einer fortlaufenden Schicht von Klebstoff auf der ersten Hauptoberfläche der Substratschicht.

12. Das Verfahren nach Anspruch 11, wobei das Modifizieren der Substratschicht durch Schneiden einer Mehrzahl von Schnitten in die Substratschicht das Schneiden mit einem Messer, einer Klinge, einem Wasserstrahl oder einem Laserstrahl aufweist.

13. Das Verfahren nach Anspruch 11, wobei die fortlaufende Schicht von Klebstoff auf der ersten Hauptoberfläche der Substratschicht vor dem Modifizieren der Substratschicht ausgebildet wird.

14. Das Verfahren nach Anspruch 13, wobei die Mehrzahl von Schnitten ebenso in der Klebeschicht vorhanden ist.

## Revendications

1. Articles adhésifs comprenant :
une couche continue d'adhésif avec une première surface principale et une seconde surface principale ;
et
une couche de substrat avec une première surface principale et la seconde surface principale, dans lesquels
la première surface principale de la couche de substrat est en contact avec la seconde surface principale de la couche d'adhésif, et
dans lesquels la couche de substrat comprend une pluralité de découpes, dans lesquels la pluralité de découpes sont des ouvertures étroites pratiquées dans la couche de substrat par pénétration d'un outil de découpe de telle sorte qu'aucun matériau n'est retiré de la couche de substrat par le processus de découpe,
dans lesquels la pluralité de découpes est disposée dans un motif, et dans lesquels les découpes sont des espaces préparés par découpe ou refente, mais
les espaces ne sont pas visibles à l'œil nu et un rapport entre une longueur et une largeur pour les découpes est supérieur à 1000 lorsque l'article adhésif est dans un état non contraint, et
au moins certaines des découpes deviennent des espaces qui sont visibles à l'œil nu, le rapport entre la longueur et la largeur des découpes diminue, et les espaces formant des orifices à travers lesquels passe la lumière, lorsque l'article adhésif est dans un état contraint.

2. Article adhésif selon la revendication 1, dans lequel au moins certains des espaces visibles à l'œil nu lorsque l'article adhésif est dans un état contraint fournissent des orifices à travers lesquels on peut voir à travers l'article adhésif.

3. Article adhésif selon la revendication 1, dans lequel la pluralité de découpes est disposée dans un motif le long d'un axe ou plus d'un axe.

4. Article adhésif selon la revendication 1, dans lequel l'adhésif comprend un adhésif en gel ou un adhésif sensible à la pression.

5. Article adhésif selon la revendication 1, dans lequel la couche de substrat comprend un film thermoplastique de polyoléfine, de polyuréthane, de polyester, ou de polyéther-amide séquencé.

6. Article adhésif selon la revendication 1, dans lequel l'état contraint comprend un étirement, une flexion ou une combinaison de ceux-ci.

7. Article adhésif selon la revendication 1, dans lequel lorsque l'article adhésif est dans un état contraint, l'article adhésif a des propriétés optiques différentes des propriétés optiques de l'article adhésif dans l'état non contraint.

8. Article adhésif selon la revendication 1, dans lequel la couche de substrat a un module d'élasticité effective plus faible dans au moins un axe qu'une couche de substrat identique sans la pluralité de découpes.

9. Article adhésif selon la revendication 1, comprenant en outre un dispositif, dans lequel le dispositif est fixé à la seconde surface principale de la couche de substrat, dans lequel le dispositif comprend un moniteur ou un capteur.

10. Construction médicale comprenant :
un article adhésif tel que défini dans l'une quelconque des revendications précédentes, pouvant être fixé à une surface comprenant de la peau de mammifère, dans laquelle l'article adhésif comprend :
une couche continue d'adhésif avec une première surface principale et une seconde surface principale ; et
une couche de substrat avec une première surface principale et la seconde surface principale, dans laquelle la première surface principale de la couche de substrat est en contact avec la seconde surface principale de la couche d'adhésif, et dans laquelle la couche de substrat comprend une pluralité de découpes, dans laquelle la pluralité de découpes sont des ouvertures étroites pratiquées dans la couche de substrat par pénétration d'un outil de découpe de telle sorte qu'aucun matériau n'est retiré de la couche de substrat par le processus de découpe, dans laquelle la pluralité de découpes est disposée dans un motif, et dans laquelle les découpes sont des espaces préparés par découpe ou refente, mais
au moins certains des espaces ne sont pas visibles à l'œil nu et un rapport entre une longueur et une largeur pour les découpes est supérieur à 1000 lorsque l'article adhésif est dans un premier état, et
au moins certains des espaces qui ne sont pas visibles à l'œil nu dans le premier état deviennent des espaces qui sont visibles à l'œil nu, les espaces formant des orifices à travers lesquels la lumière passe, et le rapport entre la longueur et la largeur des découpes diminue lorsque l'article adhésif est dans un second état, dans laquelle
le second état est un état de contrainte plus élevée de flexion, d'étirement ou d'une combinaison de ceux-ci, et dans laquelle
la première surface principale de la couche d'adhésif est conçue pour entrer en contact avec la surface comprenant une peau de mammifère.

11. Procédé de préparation d'un article adhésif tel que défini dans l'une quelconque des revendications précédentes comprenant :
la fourniture d'une couche de substrat avec une première surface principale et une seconde surface principale ;
la modification de la couche de substrat en découpant une pluralité de découpes dans la couche de substrat sans retirer de matériau de la couche de substrat, de telle sorte que
dans un état non contraint, les découpes sont des espaces, mais les espaces ne sont pas visibles à l'œil nu et un rapport entre une longueur et une largeur pour les découpes est supérieur à 1000, mais
dans un état contraint, au moins certains des espaces deviennent visibles à l'œil nu, le rapport diminue et les espaces forment des orifices à travers lesquels la lumière passe ;
la fourniture d'un adhésif ; et
la formation d'une couche continue d'adhésif sur la première surface principale de la couche de substrat.

12. Procédé selon la revendication 11, dans lequel la modification de la couche de substrat en découpant une pluralité de découpes dans la couche de substrat comprend la découpe avec un couteau, une lame, un jet d'eau ou un faisceau laser.

13. Procédé selon la revendication 11, dans lequel la couche continue d'adhésif sur la première surface principale de la couche de substrat est formée avant la modification de la couche de substrat.

14. Procédé selon la revendication 13, dans lequel la pluralité de découpes est également présente dans la couche d'adhésif.
